Europäisches Patentamt

(19) European Patent Office (11) Publication number: **0 114 677**

Office européen des brevets **B1**

(12) **EUROPEAN PATENT SPECIFICATION**

(45) Date of publication of patent specification: **03.05.89** (51) Int. Cl.⁴: **A 61 M 25/00**

(21) Application number: **84100561.4**

(22) Date of filing: **19.01.84**

(54) Medical connector system.

(30) Priority: **24.01.83 US 460585**
**19.10.83 US 543248**

(43) Date of publication of application:
**01.08.84 Bulletin 84/31**

(45) Publication of the grant of the patent:
**03.05.89 Bulletin 89/18**

(84) Designated Contracting States:
**DE FR GB IT**

(56) References cited:
**US-A-3 502 097**
**US-A-3 986 508**
**US-A-4 338 933**

(73) Proprietor: **ICU Medical, Inc.**
**23436 Madero Street**
**Mission Viejo, CA 92692 (US)**

(72) Inventor: **Lopez, George A.**
**3731 Seascape Drive**
**Huntington Beach, CA 92649 (US)**
Inventor: **Laul, Virgil R.**
**33601 Via Corvalian**
**Dana Point, CA 92629 (US)**

(74) Representative: **Altenburg, Udo, Dipl.-Phys.**
**et al**
**Patent- und Rechtsanwälte Bardehle-**
**Pagenberg-Dost-Altenburg & Partner Postfach**
**86 06 20**
**D-8000 München 86 (DE)**

Courier Press, Leamington Spa, England.

**Description**

Background of the invention
1. Field of the invention
This invention relates to connector systems used in the treatment of the injured or sick, and in particular to devices for intravenously introducing medication into a patient in a safe, convenient way.

2. Discussion of prior art
It is a common practice in treating patients, particularly patients who must be cared for under emergency conditions, with medication introduced into the patient intravenously. An intravenous solution, commonly referred to as the parenteral liquid, is fed from a container supplying this liquid through tubing via a needle which has been inserted into the patient's vein. The needle is taped securely to the patient's arm and is not likely to pull from the patient's arm if the patient moves. Medication needed to sustain the life of the patient, for example, drugs which maintain the blood pressure of the patient at the desired level, are added to the parenteral liquid. The conventional practice is to insert a needle into a sealed entry port in a connector through which the parenteral liquid flows. The way the needle is currently inserted into the sealed port, however, permits the needle to be pulled loose from the seal relatively easily. This present a problem which, though recognized by the manufacturers of conventional intravenous type medical devices, has not as yet been adequately solved. The accidental removal of the needle from the sealed port can have very serious consequences and could even lead to the death of the patient being treated.

Another problem with treating patients is infection. All too often a patient's life is seriously endangered by bacteria gaining entry into a patient's system, infecting the patient. In a vast number of cases it is unknown how the bacteria gain entry. We have observed conditions in hospitals and identified that one likely way the bacteria gain entry is by contamination of the needle inserted into the sealed entry port. This happens when the attendant notices that the needle has pulled loose and simply reinserts it even through it may now have on its surface bateria picked up by direct contact with, for example, the patient's bedding.

In a connector system known from US—A—3 502 097 an axially aligned hypodermic needle affixed to a valve body has one end extending into open communication with the interior of the valve body and its other or pointed end extending through a screw plug and self-sealing disk into a duct.

US—A—3 986 508 discloses a medical connector composed of a female part and a male part. Within the female part is a female septum that can be of a resilient polymeric material adapted to be punctured by the end of a hollow needle which extends within the female part, and the male part contains a further septum adapted to be pierced by said needle as the male part enters the female part. A bayonet-type clasp holds the male and female parts together.

It has been recognized that the above situation presents a serious health hazard to patients, and an economical, convenient and safe medical connector system as defined in claim 1, has now been provided according to the invention which is useful in treating patients. In addition to having utility for administering medication intravenously, the connector system of the present invention may be employed in a wide variety of applications where it is desirable to minimize bacterial infection. For example, it may be used with catheters or chest tubes.

In intravenous systems, it includes a feeding system through which the parenteral liquid flows into the patient intravenously. The feeding system has a conduit with a port therein, including seal means which close the port. The seal means is adapted to be penetrated by a needle which is connected to a source of the medication. According to our invention, a cap member is secured to the port, and this cap member carries the needle which penetrates the seal means. Since the cap member is secured to the port, movement of the patient does not result in removal of the needle from the seal means. The needle is also mounted within a chamber or cavity in the cap member in a way which avoids or reduces the likelihood of contamination. Furthermore, the interior walls of the cap engaging the exterior walls of the mating conduit provide a guideway that directs the needle into the center of the seal means to ensure that the needle does not scrape against the inside walls of the conduit. Particles scraped from the inside conduit wall could make their way into the patient's blood stream and result in death. This potentially lethal condition is inherent in the design of certain prior art devices, but the connector system of this invention with its mating conduit wall design so directs the needle to avoid scraping against the inside connector walls.

The connector system of this invention has several advantages. First, it is easy to manufacture and convenient to use. Secondly, and most importantly, it provides a safe way for administering medication intravenously to a patient, because (a) the cap is held securely in position, so that the needle cannot be jarred loose by movement of the patient, (b) the cap is designed to guide the needle so that it does not scrape against the inside of the conduit walls, and (c) the connector system is designed to minimize the likelihood of contamination of the needle carried by the cap member.

Brief description of the drawing
The features of the present invention can best be understood, together with the advantages discussed above and other advantages, by reference to the following description taken in connection with the drawing wherein like numerals indicate like parts.

Figure 1 is a schematic view illustrating administering medication intravenously to a patient in accordance with conventional practice.

Figure 2 is a cross-sectional view of a Y-type connector for introducing parenteral liquid and medication intravenously to the patient as shown in Figure 1.

Figure 3 is a perspective view of a connector system with some basic principles of the present invention.

Figure 4 is a cross-sectional view of the connector system of the present invention taken along line 4—4 of Figure 3.

Figure 5 is a perspective view of an embodiment of the connector system of the present invention.

Figure 6 is a cross-sectional view of the connector system of the present invention taken along line 6—6 of Figure 5.

Detailed description of the drawing

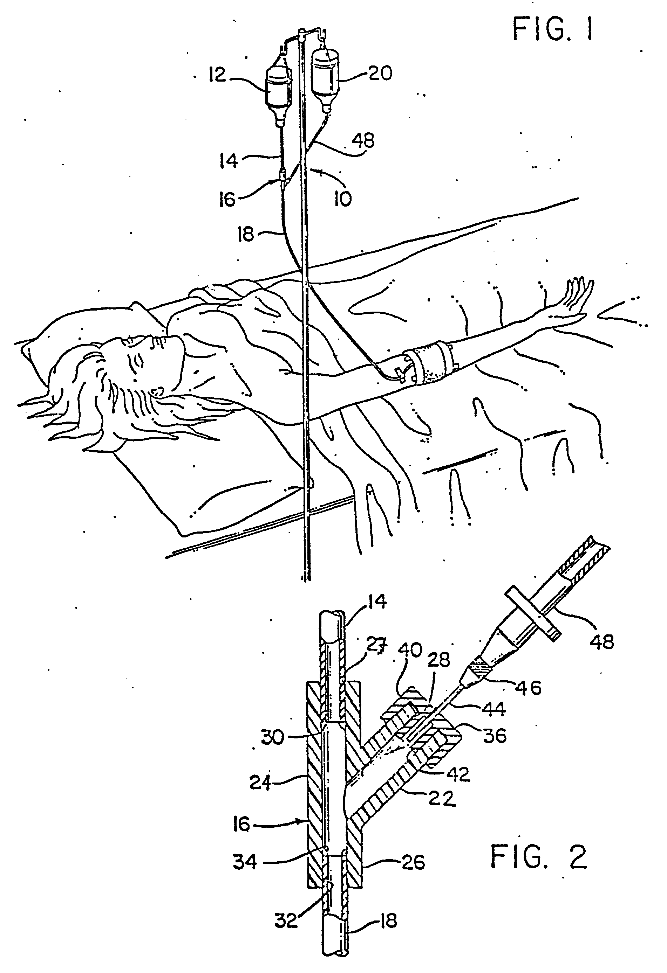

As shown in Figures 1 and 2, parenteral liquid is introduced into a patient intravenously via a feeding system 10. The feeding system 10 includes a container 12 for the parenteral liquid, a tube 14 extending from the container and connected to a Y-conector 16, and a tube 18 from the Y-connector to a needle (not shown) inserted into a vein of the patient. The needle is taped to the patient so that movement of the patient will not result in the needle being pulled from the patient's vein.

As best illustrated in Figure 2, medication from container 20 is introduced into the parenteral liquid flowing through the feeding system 10 at the Y-connector 16. This Y-connector 16 consists of two tubular conduits 22 and 24 which merge into a third tubular conduit 26. The tubing 14 from the container 12 of parenteral liquid is inserted into the inlet port 27 of the conduit 22 and secured in position, for example, by an adhesive which bonds the external surface of this tube to the internal wall surface of the conduit. There is a stop 30 which limits the extent to which this tube 14 can be inserted into the conduit. In a similar fashion, the tube 18 is secured to the outlet port 32 of the Y-connector. This tube 18 is inserted into the outlet port 32 until it abuts a stop 34 in the internal wall of the conduit. This tube 18 is then secured by an adhesive to the internal wall of the conduit 26. The branch conduit 24 has a latex seal 36 at its inlet port 28 which seals this port. Consequently, bacteria cannot enter the Y-connector 16 via the inlet port 28, because of the seal 36. This seal 36 is of conventional design and includes coaxial annular aprons 40 and 42 which fit over the conduit wall and grip the external and internal wall surfaces to hold the seal securely to the conduit 22.

The medication is introduced into the parenteral liquid flowing through the Y-connector 16 by a needle 44 which is inserted through the central part of the seal 36 into the branch conduit 22. This needle 44 is connected by a suitable connector 46 to a tube 48 which is connected to the container 20 (Figure 1) for the medication. As parenteral liquid flows through the Y-connector 16 into the inlet port 27 and out the outlet port 32, the medication is drawn into this stream of parenteral liquid, flowing from the container 20 via the tube 48 and through the open end of the needle 44 into the parenteral liquid.

The problem with the conventional device shown in Figure 2 is that if the patient moves, for example, rolls or moves his or her arm, the needle 44 may be pulled from the seal 36. If this occurs, the latex seal 36 has sufficient resiliency to close off the hole in the seal produced by the needle 44. The parenteral liquid will continue to flow into the patient's system, but the necessary medication is no longer being introduced into it. The consequences of this condition are very grave and, if this condition is unnoticed by an attendant, it could result in the death of the patient or serious complications in the patient's treatment. Even if the attendant notices that the needle 44 has been removed from the seal 36 and reinserts it into the seal, it is possible that the needle has been contaminated with bacteria. Consequently, the use of such a contaminated needle 44 is unacceptable.

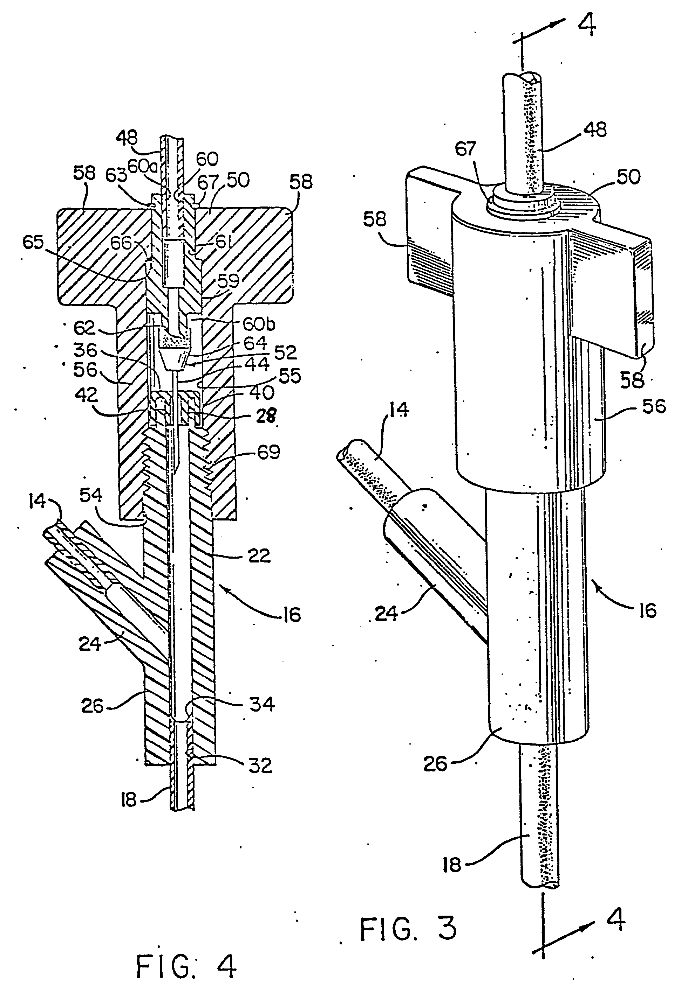

In accordance with the present invention, as illustrated in Figures 3 and 4, the needle 44 is secured to the Y-connector 16 so that movement of the patient does not result in the needle being pulled from the seal 36. The parenteral liquid is introduced via the conduit 24 and the conduit 22 is designed to receive the seal 36, with a cap member 50 carrying the needle 44 being secured to the conduit 22 so that the cap member covers the inlet port 28 and the needle penetrates the seal covering the port.

The function of the cap member 50 is threefold: First, it secures the needle 44 in position so that movement of the patient will not result in it being removed from the seal 36. Secondly, the cap member 50 surrounds the needle 44 and provides a cavity 52 in which the needle 44 is lodged so that it does not project beyond the open end 54 of the cavity. Because the needle 44 is so lodged within the cavity 52, if the attendant did, for example, lay the cap member on the patient's bed, the needle would not come into direct contact with the bed which might be infested with harmful bacteria. Thus this arrangement of the needle 44 deep within the cavity in the cap member provides additional protection for the patient. Third, the cap member 50 in coacting with the exterior wall of conduit 22 guides the needle into the center of the seal 36. Consequently, the needle does not scrape the inside wall of conduit 22 so that particles of plastic are not introduced into the patient's circulatory system. Such particles could cause death.

The cap member 50 comprises a cylindrical connector section 56 having a hollow interior forming the chamber or cavity 52. The needle, being disposed lengthwise along the longitudinal axis of the cavity, is centrally located within the cavity. Near the end 54 the interior walls 55 of the connector section 56 are threaded. As the cap member 50 is screwed onto the conduit 22, the interior cavity wall 55, sliding over the exterior surface of the conduit, serve to guide the needle 44 so that it penetrates the center of the seal. Thus, the cap member 50 and conduit 22 mate in a male-female relationship, with the needle always being housed safely within the center of the cavity in an unexposed condition. In this embodiment the cap member serves as the female component. To further insure that the needle 44 penetrates the center of the seal 36, the threads 69 could be lowered further below the seal so that the cap member would fit telescopically over the conduit 22 and then be screwed into position.

The top of the cap member 50 has a pair of outwardly extending wings 58 which facilitate screwing the cap member to the conduit 22. A spindle 59 is received within an opening 61 within the cap member 50. The body of the spindle 59 has a cylindrical neck section having a groove 63 in an end which protrudes from the opening 61. The cylindrical body expands outwardly slightly to provide a shoulder 65 which engages a stop 66 when the spindle 59 is placed in the opening, and a TRU seal C-ring 67 is received in the groove 63 to hold the spindle in position but allowing the cap member to revolve about the spindle as it is screwed onto the Y-connector 16.

Along the longitudinal axis of the spindle 59 is a passageway 60. The tube 48 from the container 20 containing the medication is inserted into the one end 60a of the passageway 60 and bonded to the internal surface of this passageway, for example, by means of an adhesive. The other end 60b of the passageway terminates in a threaded connector section 62 to which the needle 44 is secured. This needle has an adapter 64 which has an internal thread which engages the threads of the connector section 62. The hollow needle 44 extends outwardly from this adapter 64 and penetrates the seal 36 as the cap member 50 is secured to the conduit 22 by screwing it onto the conduit 22 to engage threads 69 on the external surface of the conduit just below the seal 36. Thus the needle 44 is held secure to the Y-connector 16, penetrating the center of the seal 36 with its point safely displaced away from the inside wall 55 of the conduit 22.

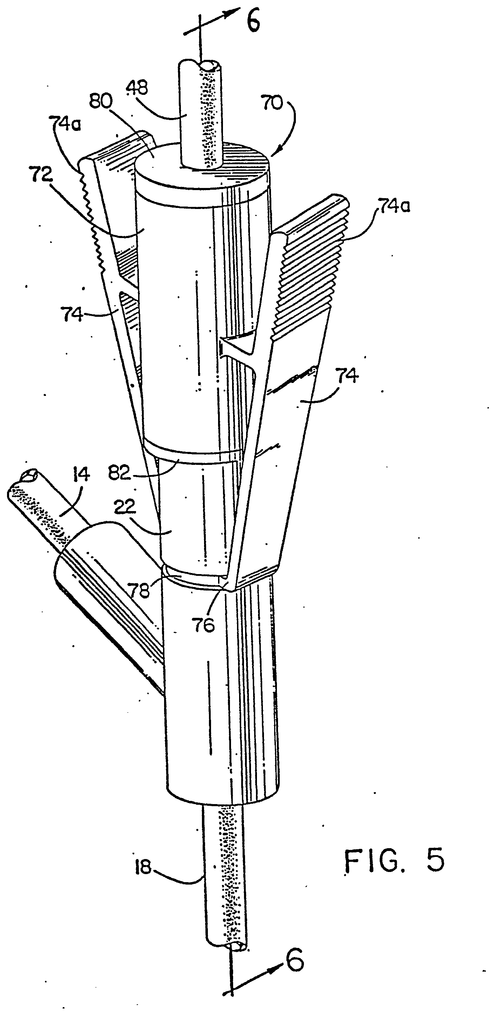

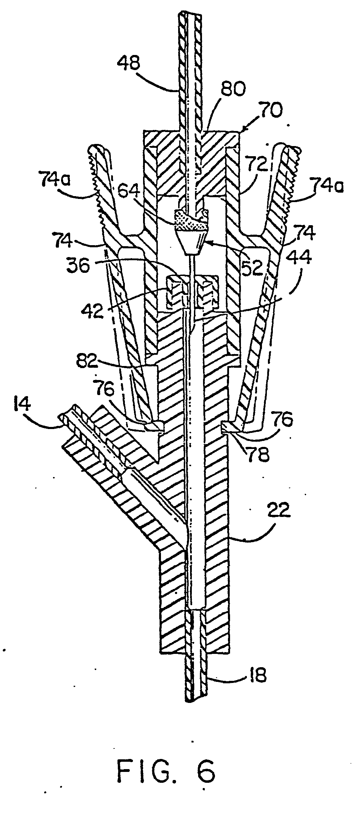

As shown in Figures 5 and 6, an alternate embodiment of the present invention is shown wherein the cap member is simply snapped on to the Y-connector 16, thereby eliminating the necessity of using a threaded cap member and threaded Y-connector. In accordance with this embodiment of the invention, the cap member 70 includes a hollow cylindrical element 72 which carries on its exterior two hingedly mounted clips 74 which have catch tips 76 which snap into a groove 78 in the external wall of the conduit 22. A plug assembly 80 carries the tubing 48 and the needle 44, which is mounted on an adapter 64 such as shown in Figure 4. This plug assembly 80 is glued or otherwise bonded to the open end of the cylindrical member 72.

To attach the cap member 70, one simply slips the cap 70 over the conduit 22. The clips 74 bend outwardly slightly and when the catch tips 76 of the clips are opposite the groove 78, the clips snap in place as shown in solid lines in Figure 6. In accordance with one of the features of this invention, the centrally mounted needle 44 is guided into the center of the seal 36 by the cap member 70, which, like a telescope, slides over the tubular conduit 22. There is a shoulder 82 which serves as a stop to limit the movement of the cap member. This shoulder 82 brings the catch tips 76 of the clips 48 into registration with the groove 78 in the conduit 22 and, because of the internal bias due to the resiliency of the material from which these clips are made, they snap into a locking position, locking the cap member to the conduit. The cap member 70 including clips 74 are made from, for example, Nylon, which is a material having the desired resiliency. To release the cap member from the Y-connector 16, the clips 74 are simply depressed and the cap member 70 is removed from the Y-connector.

As will be appreciated from the above description, there is inherent in the cap member 70 two functions in a single structure. Namely, the cap member 70 provides the cavity 52 which guards the needle 44 against contamination and guides the needle into the center of the seal 36, away from the inside wall of the conduit 22. Thus, the attendant may conveniently and safely attach and detach the cap member, without any extra steps or risk to the patient. Because of this feature, this invention may be used under normal working conditions without creating any additional work for the attendant, while substantially reducing the likelihood of harm to the patient due to carelessness.

The above description presents the best mode contemplated of carrying out the present invention. This invention is, however, susceptible to modifications and alternate constructions from the embodiments shown in the drawing and described above. Consequently, it is not the intention to limit this invention to the particular embodiments disclosed. On the contrary, the intention is to cover all modifications and alternate constructions falling within the scope of the invention as expressed in the appended claims.

## Claims

1. System (10) for feeding medication from a remote source (20) through tubing means (48, 18) having one end for securely connecting to and in communication with a patient and including port means (28) at a point upstream from said one end,

a) said port means (28) being formed by a first tubular wall element (22) having an open end sealed by a sealing means (36) and being connected to a cap member (50; 70), with the medication being injected through said port means (28) by a needle (44) that is in communication with said source (20) of medication and which is inserted into and through said sealing means (36) so that the tip of said needle (44) penetrates said sealing means (36) and medication flows into said tubing means (48, 18),

b) said sealing means (36) being of the self-sealing type so that holes produced in said sealing means (36) by repeated penetration therethrough by said needle (44) are closed off each time said needle (44) is withdrawn from said sealing means (36),

c) said cap member (50; 70) having a cavity (52) therein formed by a second tubular wall element (56; 72) which provides an open end (54) with said needle (44) being disposed lengthwise along the longitudinal axis of said cavity (52), with said tip of said needle (44) being displaced inwardly from said open end (54), a sufficient distance to recess said needle (54) deep within said cavity (52) so that it will unlikely be contaminated,

d) said first tubular wall element (22) of said port means (28) being adapted to pass through said open end (54) upon insertion of said port means (28) into said cavity (52) of said cap member (50, 70) with said first and second wall elements (22; 56; 72) being coaxially aligned and engaging in a male-female mating relationship as the port means (28) enters said open end (54) of said cavity (52), with the interior surface (55) of said second wall element (56; 72) sliding over the exterior surface of said first wall element (22), said surfaces serving as guide means for directing said needle (44) into the central portion of said sealing means (36) so that said tip of said needle (44) does not scrape particles from the inside surface of said first wall element (22), and

e) said cap member (70, 72) being of the snap-on type wherein said cap member (70, 72) has clip means (74) attached thereto for detachably connecting said cap member (70, 72) to said port means (28).

2. System according to claim 1, characterized in that said cap member (70, 72) carries on its exterior two hingedly mounted clips (74) which have catch tips (76) which snap into a groove (78) on the exterior of said first wall element (22) of said port means (28).

3. System according to claim 1 or 2, characterized in that said needle (44) is removably attached to the central portion of the closed rear end of said cap member (50; 70).

**Patentansprüche**

1. System (10) zum Fördern von Medizin von einer entfernten Quelle (20) durch Leitungseinrichtungen (48, 18), welche ein Ende aufweisen zum sicheren Verbinden mit und in Verbindung mit einem Patienten und welche an einem Punkt stromaufwärts von dem einen Ende Anschlußeinrichtungen (28) aufweisen,

a) wobei die Anschlußeinrichtungen (28) gebildet werden durch ein erstes rohrförmiges Wandelement (22), dessen eines offenes Ende durch eine Abdichtungseinrichtung (36) abgedichtet ist und mit einem Kappenbauteil (50; 70) verbunden ist, wobei die Medizin durch die Anschlußeinrichtung (28) durch eine Nadel (44) injiziert wird, welche in Verbindung mit der Medizinquelle (20) steht und welche in und durch die Abdichtungseinrichtung (36) eingeführt wird, so daß die Spitze der Nadel (44) die Abdichtungseinrichtung (36) durchdringt und Medizin in die Leitungseinrichtung (48, 18) strömt,

b) wobei die Abdichtungseinrichtung (36) von selbstabdichtender Art ist, so daß Löcher, welche in der Abdichtungseinrichtung (36) durch wiederholtes Durchdringen der Nadel (44) erzeugt werden, jedesmal geschlossen werden, wenn die Nadel (44) aus der Abdichtungseinrichtung (36) zurückgezogen wird,

c) wobei das Kappenbauteil (50; 70) einen Hohlraum (52) aufweist, geformt durch ein zweites rohrförmiges Wandelement (56; 72), welches ein offenes Ende (54) hat, wobei die Nadel (44) in Längsrichtung längs der Längsachse des Hohraums (52) angeordnet ist, wobei die Spitze der Nadel (44) nach innen versetzt vom offenen Ende (44) um einen ausreichenden Abstand angeordnet ist, um die Nadel (44) tief innerhalb des Hohlraum (52) zu versenken, so daß sie höchst umwahrscheinlich beschmutzt wird,

d) wobei das erste rohrförmige Wandelement (22) der Anschlußeinrichtung (28) durch das offene Ende (54) bei Einführung der Anschlußeinrichtung (28) in den Hohlraum (52) des Kappenbauteils (50, 70) hindurchtreten kann, wobei das erste und das zweite Wandelement (22; 56; 72) koaxial ausgerichtet sind und in einer männlichen-weiblichen Paßbeziehung in Eingriff stehen, wenn die Anschlußeinrichtung (28) in das offene Ende (54) des Hohlraums (52) eintritt, wobei die innere Oberfläche (55) des zweiten Wandelementes (56; 72) über die äußere Oberfläche des ersten Wandelementes (22) gleitet, wobei die Oberflächen als Führungseinrichtung dienen zur Ausrichtung der Nadel (44) in den mittleren Abschnitt der Abdichtungseinrichtung (36), so daß die Spitze der Nadel (44) keine Partikel von der Innenoberfläche des ersten Wandelementes (22) abkratzt, und

e) wobei das Kappenbauteil (70, 72) eine Aufschnappbauart hat, wobei das Kappenbauteil (70, 72) Haltereinrichtungen (74) aufweist, welche an diesem befestigt sind zur lösbaren Verbindung des Kappenbauteils (70, 72) mit der Anschlußeinrichtung (28).

2. System nach Anspruch 1, dadurch gekennzeichnet, daß das Kappenbauteil (70, 72) auf seiner Außenseite zwei gelenkartig gelagerte Halter (74) trägt, welche Greifspitzen (76) aufweisen, welche in eine Nut (78) auf der Außenseite des ersten Wandelementes (22) der Anschlußeinrichtung (28) schnappen.

3. System nach Anspruch 1 oder 2, dadurch gekennzeichnet, daß die Nadel (44) entfernbar am mittleren Abschnitt des geschlossenen rückwärtigen Endes des Kappenbauteils (50; 70) befestigt ist.

**Revendications**

1. Ensemble (10) d'alimentation en médicament provenant d'une source distante (20) par l'intermédiaire d'un tube (48, 18) dont une première extrémité est fixée à un patient avec lequel il communique, et comprenant un orifice (28) à un emplacement qui se trouve en amont de cette extrémité,

a) l'orifice (28) étant formé par un premier élément (22) de paroi tubulaire ayant une extrémité ouverte qui peut être fermée par un dispositif de scellement (36) et étant raccordé à un capuchon (50; 70), le médicament étant injecté par l'intermédiaire de l'orifice (28) à l'aide d'une aiguille (44) qui communique avec la source (20) de médicament et qui est introduite dans le dispositif de scellement (36) et à travers celui-ci de manière que le bout de l'aiguille (44) traverse le dispositif de scellement (36) et que le médicament s'écoule dans le tube (48, 18),

b) le dispositif de scellement (36) étant du type à scellement automatique, tel que les trous formés dans le dispositif de scellement (36) par pénétration répétée de l'aiguille (44) se referment chaque fois que l'aiguille (44) est retirée du dispositif de scellement (36),

c) le capuchon (50; 70) ayant une cavité (52) qui y est formée par un second élément (56; 72) de paroi tubulaire qui forme une extrémité ouverte (54), l'aiguille (44) étant disposée longitudinalement suivant l'axe longitudinal de la cavité (52), le bout de l'aiguille (44) étant décalé vers l'intérieur, par rapport à l'extrémité ouverte (54), d'une distance suffisante pour que l'aiguille (54) soit profondément en retrait dans la cavité (52) et présente ainsi peu de risque de contamination,

d) le premier élément (22) de paroi tubulaire de l'orifice (28) étant destiné à être disposé dans l'extrémité ouverte (54) après introduction du dispositif formant l'orifice (28) dans la cavité (52) du capuchon (50, 70), le premier et le second élément (22; 56; 72) de paroi étant alignés coaxialement et coopérant par un raccord mâle-femelle lorsque le dispositif à orifice (28) pénètre à l'extrémité ouverte (54) de la cavité (52), la surface (55) du second élément (56; 72) de paroi glissant sur la surface externe du premier élément (22) de paroi, les surfaces constituant un dispositif de guidage destiné à diriger l'aiguille (44) dans la partie centrale du dispositif de scellement (36) afin que le bout de l'aiguille (44) n'arrache pas de particules de la surface interne du premier élément (22) de paroi, et

e) le capuchon (70, 72) étant du type à enclenchement élastique tel que le capuchon (70, 72) comporte des pinces (74) qui y sont fixées et qui assurent le raccordement temporaire du capuchon (70, 72) au dispositif à orifice (28).

2. Ensemble selon la revendication 1, caractérisé en ce que le capuchon (70, 72) porte, à l'extérieur, deux pinces articulées (74) qui ont des bouts d'accrochage (76) qui s'enclenchent élastiquement dans une gorge (78) formée à l'extérieur du premier élément (22) de paroi du dispositif à orifice (28).

3. Ensemble selon la revendication 1 ou 2, caractérisé en ce que l'aiguille (44) est fixée de façon amovible sur la partie central de l'extrémité arrière fermée du capuchon (50; 70).

FIG. 1

FIG. 2

FIG. 4

FIG. 3

FIG. 5

FIG. 6